# EUROPÄISCHE PATENTANMELDUNG

(11) **EP 1 459 717 A1**
(43) Veröffentlichungstag der Anmeldung: **22.09.2004**
(21) Anmeldenummer: 04005823.2
(22) Anmeldetag: 11.03.2004
(51) Int. Cl.: A61F 13/02, A61F 15/00

(54) **Wundversorgungsprodukt mit Abziehhilfe und Verfahren zur Herstellung desselben**

(30) Priorität: 20.03.2003 DE 10312452
(71) Anmelder: Lohmann & Rauscher GmbH & Co. KG, 56567 Neuwied (DE)
(72) Erfinder: Albus, Sascha, 56581 Melsbach (DE); Thewalt, Joachim, 56581 Melsbach (DE); Grothaus, Heike, 65681 Kurtscheid (DE); Kaiser, Martin, 56567 Neuwied (DE); Hahn, Andreas, 56566 Neuwied (DE); Slupik, Gabriel, 57614 Woldert (DE)
(74) Vertreter: Patentanwälte Leinweber & Zimmermann

(57) **Zusammenfassung**

Die Erfindung betrifft ein Wundversorgungsprodukt mit einem eine haftende Klebefläche aufweisenden bahnförmigen Wundversorgungselement und einer die Klebefläche abdeckenden Abdeckanordnung mit mindestens zwei Abdeckelementen, von denen mindestens einem eine das Lösen des Abdeckelementes von dem bahnförmigen Wundversorgungselement erleichternde Abziehhilfe zugeordnet ist, wobei die Abdeckelemente durch eine Trennlinie voneinander getrennt sind und mindestens eine Abziehhilfe sich ausgehend von der Trennlinie das entsprechende Abdeckelement überlappend in eine dem benachbarten Abdeckelement entgegengesetzte Richtung erstreckt.

## Beschreibung

Die Erfindung betrifft ein Wundversorgungsprodukt mit einem eine haftende Klebefläche aufweisenden bahnförmigen Wundversorgungselement und einer die Klebefläche abdeckenden Abdeckanordnung mit mindestens zwei Abdeckelementen, von denen mindestens einem eine das Lösen des Abdeckelementes von dem bahnförmigen Element erleichternde Abziehhilfe zugeordnet ist sowie ein Verfahren zum Herstellen derartiger Wundversorgungsprodukte. Derartige Wundversorgungsprodukte werden in Form von Rollen mit verschiedenen Breiten, wie etwa 107 mm und 157 mm, hergestellt. Sie werden zur Prophylaxe (u.a. Dekubitus), Fixierung und Abdeckung in der Wundversorgung eingesetzt. Dazu werden die erforderlichen Produktlängen von der Rolle abgeschnitten. Dann werden die Abdeckelemente unter Verwendung der Abziehhilfe von der Klebefläche entfernt und schließlich wird das Wundversorgungselement mit seiner Klebefläche appliziert. Bei herkömmlichen Wundversorgungsprodukten der vorstehend beschriebenen Art umfaßt die Abdeckanordnung zwei einander überlappende Abdeckelemente, von denen jede eine Abziehhilfe in Form eines auf sich selbst zurückgefalteten Endstreifens des Abdeckelementes umfaßt. Derartige Wundversorgungsprodukte bereiten nicht nur bei der Applikation, sondern auch bei der Herstellung Probleme.

Angesichts der vorstehend beschriebenen Probleme im Stand der Technik liegt der Erfindung die Aufgabe zugrunde, ein Wundversorgungsprodukt der eingangs beschriebenen Art bereitzustellen, welches einfach herstellbar ist und dennoch eine einfache Applikation erlaubt, sowie ein zur Herstellung derartiger Wundversorgungsprodukte geeignetes Verfahren anzugeben.

Hinsichtlich des Wundversorgungsproduktes wird diese Aufgabe durch eine Weiterbildung der bekannten Wundversorgungsprodukte gelöst, die im wesentlichen dadurch gekennzeichnet ist, daß die Abdeckelemente durch eine Trennlinie voneinander getrennt sind und mindestens eine Abziehhilfe sich ausgehend von der Trennlinie das entsprechende Abdeckelement überlappend in eine dem benachbarten Abdeckelement entgegengesetzte Richtung erstreckt.

lm Rahmen der Erfindung werden also ohne gegenseitige Überlappung direkt aneinander angrenzende Abdeckelemente eingesetzt. Dadurch wird die Applikation des Wundversorgungsproduktes bzw. Wundversorgungselementes erleichtert, weil die Abdeckelemente ohne Beseitigung der gegenseitigen Überlappung von der Klebefläche des Wundversorgungselementes gelöst werden können. Darüber hinaus ist auch die Herstellung erfindungsgemäßer Wundversorgungsprodukte besonders einfach, weil keine Überlappung der Abdeckelemente im Verlauf des Herstellungsvorganges bewirkt werden muß. Dabei beruht die Erfindung auf der Erkenntnis, daß die bislang als erforderlich angesehene vollständige Abdeckung der Klebefläche durch die Abdeckanordnung, welche durch die gegenseitige Überlappung der Abdeckelemente bewirkt wurde, in vielen Fällen nicht erforderlich ist. Das gilt insbesondere für solche Fälle, bei denen das Wundversorgungsprodukt lediglich zur Fixierung und Abdeckung in der Wundversorgung eingesetzt wird. Im Hinblick auf die vereinfachte Herstellung und Handhabung kann ein im Bereich der Trennlinie entstehender schmaler freiliegender Streifen der Klebefläche in Kauf genommen werden, ohne daß die Gebrauchseigenschaften des Wundversorgungsproduktes dadurch eine wesentliche Beeinträchtigung erfahren.

Im Hinblick auf eine weitere Vereinfachung der Applikation erfindungsgemäßer Wundversorgungsprodukte hat es sich als vorteilhaft erwiesen, wenn jedem der durch die Trennlinie voneinander getrennten Abdeckelemente eine sich ausgehend von der Schnittlinie in eine dem jeweils benachbarten Abdeckelement entgegengesetzte Richtung erstrekkende Abziehhilfe zugeordnet ist. Die zuletzt beschriebene Ausführungsform der Erfindung kann besonders einfach hergestellt werden, wenn die den durch die Trennlinie voneinander getrennten Abdeckelementen zugeordneten Abziehhilfen durch die Schnittlinie voneinander getrennte über sich jeweils etwa parallel zu der Schnittlinie erstreckende und durch diese voneinander getrennte Klebestreifen an dem entsprechenden Abziehelement befestigte Abziehstreifen aufweisen. Derartige Wundversorgungsprodukte können besonders einfach ohne Faltvorgang zum Verwirklichen der Abziehhilfen hergestellt werden, indem eine Klebefläche eines bahnförmigen Elementes mit einem Abdeckstreifen abgedeckt, danach eine bahnförmige Hilfsanordnung über zwei etwa parallel zueinander verlaufende Klebestreifen an dem Abdeckstreifen befestigt wird und anschließend eine den Abdeckstreifen und die Hilfsanordnung durchsetzende Trennlinie zwischen diesen Klebestreifen gebildet wird, um so zwei getrennte Abdeckelemente mit jeweils einem daran befestigten Abziehstreifen zu bilden.

Eine weitere Vereinfachung der Applikation erfindungsgemäßer Wundversorgungsprodukte kann erreicht werden, wenn mindestens eine Abziehhilfe optisch, insbesondere farblich, und/oder haptisch von dem entsprechenden Abziehelement abgesetzt ist. In diesem Fall können die Abziehhilfen nach Trennen eines Streifens geeigneter Länge von dem Rest des aufgerollten bahnförmigen Wundversorgungsproduktes schnell erkannt und zum Applizieren des Wundversorgungselementes sicher erfaßt werden. Dabei kann die Abziehhilfe aus einem anderen Material bestehen. Insbesondere ist daran gedacht, die Abziehhilfe in Form von herkömmlichen (gefärbten) Papierstreifen zu verwirklichen und die Abdeckelemente durch Silikonpapier zu verwirklichen.

Ein ungewolltes Ablösen der Abziehelemente im Bereich der seitlichen Ränder des Wundversorgungsproduktes kann verhindert werden, wenn die Abdeckanordnung das bahnförmige Element im Bereich mindestens eines der seitlichen Ränder des Wundversorgungselementes umläuft und vorzugsweise an mindestens einem umlaufenen Rand des bahnförmigen Wundversorgungselementes ein Verstärkungselement vorgesehen ist. Dabei können die Abdeckelemente über eine die der Klebefläche abgewandte Begrenzungsfläche des Wundversorgungselementes umlaufende Stützfolie miteinander verbunden sein. Alternativ oder zusätzlich kann die Stützfolie über separate Verstärkungsstreifen mit den Abdeckelementen verbunden sein. Mit anderen Worten bedeutet das, daß die Stützfolie einstückig mit den Abdeckelementen hergestellt sein kann oder aber auch separat davon.

Nachstehend wird die Erfindung unter Bezugnahme auf die Zeichnung, auf die hinsichtlich aller erfindungswesentlichen und in der Beschreibung nicht näher herausgestellten Einzelheiten ausdrücklich verwiesen wird, erläutert. In der Zeichnung zeigt:

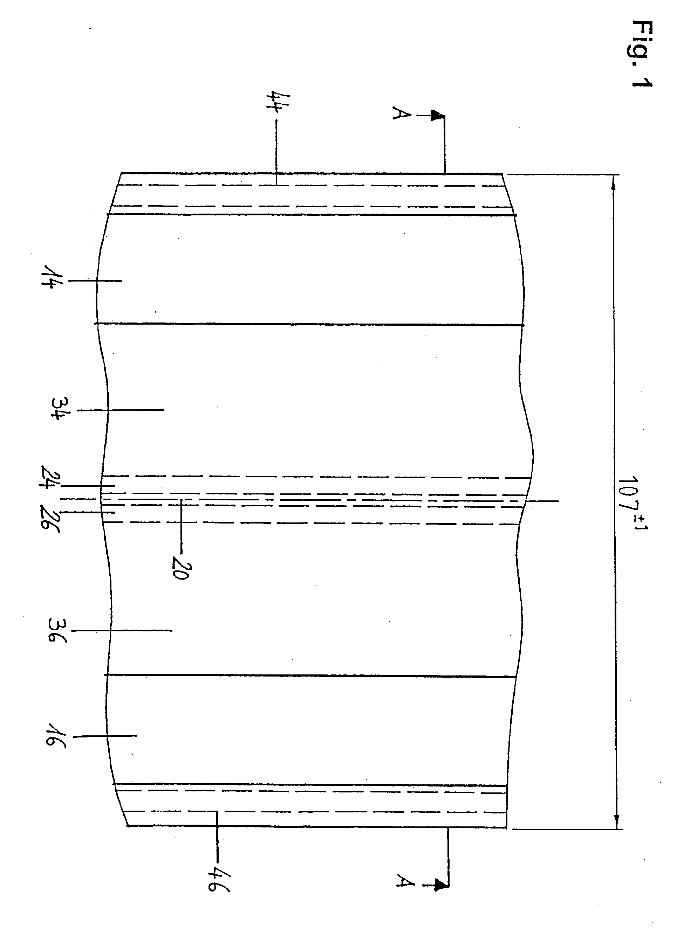

- **Fig. 1**: eine Draufsicht auf ein erfindungsgemäßes Wundversorgungsprodukt und

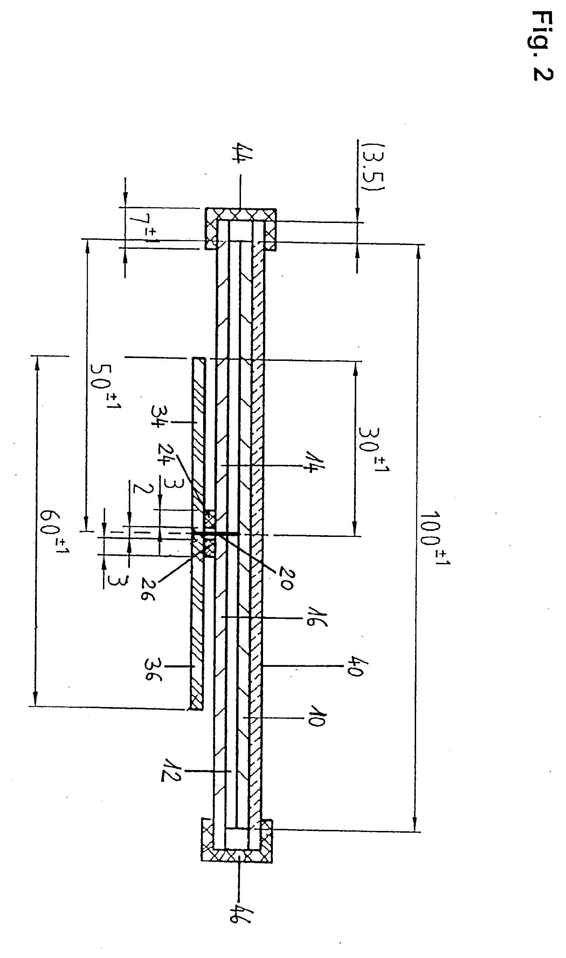

- **Fig. 2**: eine Schnittdarstellung des in Fig. 1 dargestellten Wundversorgungsproduktes längs der in Fig. 1 angegebenen Schnittebene A - A.

Das in der Zeichnung dargestellte Wundversorgungsprodukt umfaßt ein in Form eines PU-Films verwirklichtes bahnförmiges Wundversorgungselement 10. Auf einer durch eine Klebeschicht 12 gebildeten Klebefläche des Wundversorgungselementes 10 sind zwei Abdeckelemente 14 und 16 angeordnet. Die Abdeckelemente 14 und 16 sind ohne gegenseitige Überlappung durch eine Trennlinie 20 voneinander getrennt. Im Bereich der Trennlinie 20 stoßen einander zugewandte Ränder der Abdeckelemente 14 und 16 stumpf aneinander. An dem Abdeckelement 14 ist über einen Klebestreifen 24 ein Abziehstreifen 34 befestigt. An dem Abdeckelement 16 ist über einen Klebestreifen 26 ein Abziehstreifen 36 als Abziehhilfe befestigt. Die Abdeckelemente 14 und 16 bestehen aus Silikonpapier, während die Abziehstreifen 34 und 36 aus einem gefärbten Papier bestehen. Die Abdeckelemente 14 und 16 sind über die seitlichen Ränder des Wundversorgungselementes 10 umlaufende Verstärkungsstreifen mit einer Stützfolie verbunden. Die Stützfolie deckt eine der Klebefläche 12 abgewandte Begrenzungsfläche des Wundversorgungselementes 10 ab.

Zum Herstellen des in der Zeichnung dargestellten Wundversorgungsproduktes wird ein Abdeckstreifen über die Klebeschicht 10 an dem Wundversorgungselement befestigt. Dann wird eine bahnförmige Abziehhilfe über die beiden parallel zueinander verlaufenden Klebestreifen 24 und 26 an der der Klebefläche abgewandten Begrenzungsfläche des Abdeckstreifens befestigt. Anschließend werden die Abziehhilfe und die der Abdeckstreifen längs einer zwischen den Klebestreifen 24 und 26 verlaufenden Trennlinie durchgestanzt. Dabei wird jedoch darauf geachtet, daß das Wundversorgungselement 10 nicht durchtrennt wird. Auf diese Weise werden aus dem Abdeckstreifen und der Abziehhilfe die Abdeckelemente 14 und 16 sowie die Abziehstreifen 34 und 36 gebildet. Dabei kann die Anbringung der Abziehhilfe an dem Abdeckstreifen vor oder nach der Anbringung des Abdeckstreifens an der Klebefläche des Wundversorgungselementes erfolgen.

Die Erfindung ist nicht auf das anhand der Zeichnung erläuterte Ausführungsbeispiel beschränkt. Vielmehr ist auch daran gedacht, die Klebefläche des Abstützelementes durch drei oder mehr Abdeckelemente abzudecken. Ferner kann auf die Stützfolie 40 verzichtet werden. Auch ist daran gedacht, die Abziehstreifen an einer anderen Stelle an den Abdeckelementen zu befestigen. Ferner kann das erfindungsgemäße Produkt in beliebigen Längen und Breiten hergestellt werden.

## Patentansprüche

1. Wundversorgungsprodukt mit einem eine haftende Klebefläche aufweisenden bahnförmigen Wundversorgungselement und einer die Klebefläche abdeckenden Abdeckanordnung mit mindestens zwei Abdeckelementen (14, 16), von denen mindestens einem eine das Lösen des Abdeckelementes (14, 16) von dem bahnförmigen Wundversorgungselement (10) erleichternde Abziehhilfe (34, 36) zugeordnet ist, **dadurch gekennzeichnet, daß** die Abdeckelemente (14, 16) durch eine Trennlinie (20) voneinander getrennt sind und mindestens eine Abziehhilfe (34, 36) sich ausgehend von der Trennlinie (20) das entsprechende Abdeckelement (14, 16) überlappend in eine dem benachbarten Abdeckelement (14, 16) entgegengesetzte Richtung erstreckt.

2. Wundversorgungsprodukt nach Anspruch 1, **dadurch gekennzeichnet, daß** jedem der durch die Trennlinie (20) voneinander getrennten Abdeckelemente (14, 16) eine sich ausgehend von der Schnittlinie (20) in eine dem jeweils benachbarten Abdeckelement entgegengesetzte Richtung erstreckende Abziehhilfe (34, 36) zugeordnet ist.

3. Wundversorgungsprodukt nach Anspruch 1 oder 2, **dadurch gekennzeichnet, daß** mindestens eine Abziehhilfe (34, 36) einen über einen sich etwa parallel zur Trennlinie (20) erstreckenden Klebestreifen (24, 26) an dem entsprechenden Abdeckelement (14, 16) befestigten Abziehstreifen (34, 36) aufweist.

4. Wundversorgungsprodukt nach Anspruch 2 oder 3, **dadurch gekennzeichnet, daß** die den durch die Trennlinie (20) voneinander getrennten Abdeckelementen (14, 16) zugeordneten Abziehhilfen (34, 36) durch die Schnittlinie voneinander getrennte und über sich jeweils etwa parallel zu der Schnittlinie erstreckende und durch diese voneinander getrennte Klebestreifen an dem entsprechenden Abziehelement befestigte Abziehstreifen (34, 36) aufweisen.

5. Wundversorgungsprodukt nach einem der vorhergehenden Ansprüche, **dadurch gekennzeichnet, daß** mindestens eine Abziehhilfe (34, 36) optisch, insbesondere farblich, und/oder haptisch von dem entsprechenden Abdeckelement (14, 16) abgesetzt ist.

6. Wundversorgungsprodukt nach Anspruch 5, **dadurch gekennzeichnet, daß** die Abziehhilfe (34, 36) aus einem anderen Material besteht als das Abdeckelement (14, 16).

7. Wundversorgungsprodukt nach einem der vorhergehenden Ansprüche, **dadurch gekennzeichnet, daß** die Abdeckanordnung das bahnförmige Wundversorgungselement (10) umläuft und vorzugsweise an mindestens einem umlaufenden Rand des bahnförmigen Wundversorgungselementes (10) ein Verstärkungselement aufweist.

8. Verfahren zum Herstellen eines Wundversorgungsproduktes nach einem der vorhergehenden Ansprüche, bei dem eine Klebefläche eines bahnförmigen Wundversorgungselementes mit einem Abdeckstreifen abgedeckt und eine bahnförmige Hilfsanordnung auf der dem bahnförmigen Element abgewandten Begrenzungsfläche des Abdeckstreifens befestigt wird und abschließend eine den Abdeckstreifen und die Hilfsanordnung durchsetzende Trennlinie zum Herstellen von zwei getrennten Abdeckelementen mit jeweils einem daran befestigten Abziehstreifen gebildet wird.

9. Verfahren nach Anspruch 8, **dadurch gekennzeichnet, daß** die Trennlinie durch einen Stanzvorgang gebildet wird.

10. Verfahren nach Anspruch 8 oder 9, **dadurch gekennzeichnet, daß** die bahnförmige Hilfsanordnung über zwei etwa parallel zueinander verlaufende Klebestreifen an dem Abdeckstreifen befestigt und die Trennlinie zwischen diesen Klebestreifen gebildet wird.
